# EUROPEAN PATENT APPLICATION

(11) **EP 4 624 039 A1**
(43) Date of publication of application: **01.10.2025**
(21) Application number: 22966198.8
(22) Date of filing: 24.11.2022
(51) Int. Cl.: B01J 8/26, B01J 8/18, C07C 15/08

(54) **CIRCULATING FLUIDIZED BED REACTION REGENERATION DEVICE AND USE METHOD**

(71) Applicant: Dalian Institute Of Chemical Physics, Chinese Academy Of Sciences, Liaoning 116023 (CN); China Shenhua Coal to Liquid and Chemical Co., Ltd, Beijing 100011 (CN)
(72) Inventor: ZHANG, Tao, Dalian, Liaoning 116023 (CN); YE, Mao, Dalian, Liaoning 116023 (CN); YAN, Guochun, Beijing 100011 (CN); LIU, Zhongmin, Dalian, Liaoning 116023 (CN); ZHANG, Jinling, Dalian, Liaoning 116023 (CN); ZHANG, Jiming, Beijing 100011 (CN); ZHANG, Cheng, Dalian, Liaoning 116023 (CN); WEN, Liang, Beijing 100011 (CN); TANG, Hailong, Dalian, Liaoning 116023 (CN); YIN, Tian, Beijing 100011 (CN); JIA, Jinming, Dalian, Liaoning 116023 (CN); LIN, Huadong, Beijing 100011 (CN); WANG, Xiangao, Dalian, Liaoning 116023 (CN); MA, Xiangang, Dalian, Liaoning 116023 (CN); WANG, Jing, Dalian, Liaoning 116023 (CN); GAO, Mingbin, Dalian, Liaoning 116023 (CN); LI, Xue, Dalian, Liaoning 116023 (CN); XU, Shuliang, Dalian, Liaoning 116023 (CN); LI, Hua, Dalian, Liaoning 116023 (CN); LI, Chenggong, Dalian, Liaoning 116023 (CN)
(74) Representative: Bayramoglu et al.
(86) International application number: PCT/CN2022/134158
(87) International publication number: WO 2024/108507

(57) **Abstract**

The present application discloses a circulating fluidized bed reaction regeneration device and its application method. The device comprises a fluidized bed reactor (1), a fluidized bed regenerator (2) and a riser reactor (3). The fluidized bed reactor (1) is used for introducing a naphtha feedstock and a methanol feedstock, wherein the naphtha feedstock is brought into contact with a catalyst from the riser reactor (3), so as to perform a reaction to generate a BTX-containing product gas flow and a spent catalyst, and the methanol feedstock undergoes a methylation reaction with benzene and toluene in the BTX-containing product gas flow to generate p-xylene; the product gas flow is subjected to gas-solid separation, the separated product gas is conveyed to a downstream section, unconverted naphtha is returned as a feedstock to the fluidized bed reactor, part of light alkanes is returned as a feedstock to the riser reactor (3), and the spent catalyst is introduced into the fluidized bed regenerator (2); and an inlet of the riser reactor (3) is connected to the fluidized bed regenerator (2), and an outlet of the riser reactor (3) is connected to the fluidized bed reactor (1). The method comprises: preparing an aromatic hydrocarbon by using the circulating fluidized bed reaction regeneration device and a metal molecular sieve bifunctional catalyst.

## Description

### TECHNICAL FIELD

The present application relates to a fluidized bed device and a method for use thereof, and specifically relates to a circulating fluidized bed reaction-regeneration device and its application method thereof, belonging to the technical field of chemical engineering.

### BACKGROUND

Aromatics (benzene, toluene, and xylene, collectively referred to as BTX) are important organic chemical raw materials. Among them, para-xylene (PX) is the most noteworthy product in aromatics, mainly used to produce terephthalic acid (PTA), polyethylene terephthalate (PET), polybutylene terephthalate (PBT), and polytrimethylene terephthalate (PTT). In recent years, China's production and consumption of PX have continued to grow. In 2021, China's total PX imports amounted to approximately 13.65 million tons, with an external dependency of about 38%.

Naphtha catalytic reforming technology is the primary technical route for producing aromatics. The composition of naphtha is highly complex, as it serves not only as the main feedstock for catalytic reforming but also as a key raw material for ethylene production via cracking. The composition of naphtha plays a decisive role in the economic efficiency of the device. Generally, feedstock with high aromatic potential content and a suitable distillation range is favorable for catalytic reforming, whereas feedstock with high linear and branched aliphatic hydrocarbon content and low naphthene and aromatic content is suitable for ethylene cracking. To fully utilize naphtha resources and improve economic efficiency, it is necessary to separate linear and branched aliphatic hydrocarbons from naphthenes and aromatics in naphtha, with the former used as feedstock for ethylene production and the latter as feedstock for catalytic reforming devices.

Naphtha fractions have a wide distillation range, making it difficult for conventional separation methods to efficiently separate linear and branched aliphatic hydrocarbons from naphthenes and aromatics. Additionally, catalytic reforming technology struggles to convert linear and branched aliphatic hydrocarbons into aromatics. Naphtha feedstock for catalytic reforming typically requires distillation to remove light fractions (boiling below 60°C), thereby improving the aromatic potential content of the catalytic reforming feedstock. However, fractions with boiling points above 60°C still contain significant amounts of linear and branched aliphatic hydrocarbons that are difficult to convert into aromatics. Therefore, the high-selectivity conversion of linear and branched aliphatic hydrocarbons into aromatics has been a key focus and challenge in the development of naphtha-to-aromatics technology.

Due to thermodynamic equilibrium limitations, para-xylene accounts for only about 24% of the xylene mixture produced by naphtha catalytic reforming devices, necessitating further para-xylene production through isomerization-separation processes. Thus, increasing the para-xylene content in the xylene mixture is an important approach to reducing energy consumption in para-xylene production.

### SUMMARY

The naphtha molecule contains only a small amount of methyl groups (methyl/benzene ring = about 1.3 (molar ratio)). Its molecular structure determines that catalytic reforming/aromatics complex units inevitably produce large amounts of benzene as byproducts.

Methanol aromatization is an emerging process for producing aromatics. However, compared to aromatics, methanol molecules contain excess hydrogen atoms. Therefore, methanol-to-aromatics conversion inevitably yields large amounts of alkanes and hydrogen as byproducts. From the perspective of molecular structure and reaction mechanisms, methanol can provide methyl groups to aromatics, thereby increasing toluene and xylene production. This offers a new technical pathway for coupled aromatics production from naphtha and methanol.

To achieve aromatics production using naphtha and methanol as feedstocks, this application provides a circulating fluidized bed reaction-regeneration device and its application method.

The naphtha components described in the present application include C₄-C₁₂ linear aliphatic hydrocarbons, branched aliphatic hydrocarbons, cycloalkanes, and aromatics.

The aromatics described in the present application refer to benzene, toluene, and xylene, collectively referred to as BTX.

According to one aspect of the present application, a circulating fluidized bed reaction-regeneration device is provided, comprising a fluidized bed reactor, a fluidized bed regenerator and a riser reactor;
the fluidized bed reactor is configured to introduce naphtha feedstock and methanol feedstock, wherein the naphtha feedstock contacts with a catalyst from the riser reactor to produce a product gas flow containing BTX and a spent catalyst, and the methanol feedstock undergoes methylation reaction with benzene and toluene in the product gas flow containing BTX to generate para-xylene; the product gas flow undergoes gas-solid separation, with the separated product gas being delivered to downstream sections, unconverted naphtha being recycled as feedstock to the fluidized bed reactor, and partial light alkanes being recycled as feedstock to the riser reactor, while the spent catalyst is introduced into the fluidized bed regenerator;
the inlet of the riser reactor is connected to the fluidized bed regenerator, and the outlet of the riser reactor is connected to the fluidized bed reactor.

Preferably, the fluidized bed reactor comprises a reactor shell, wherein the region enclosed by the reactor shell is divided from top to bottom into a first gas-solid separation zone and a reaction zone, the reaction zone being provided with a reactor distributor comprising n sub-distributors arranged sequentially from bottom to top as the 1^{st} sub-distributor to the n^{th} sub-distributor, where n≥2 and n≤10; the 1^{st} sub-distributor is configured to introduce naphtha feedstock; and the 2^{nd} to n^{th} sub-distributors are configured to introduce methanol feedstock.

Preferably, the first gas-solid separation zone is provided with a gas-solid separation unit I, a gas-solid separation unit II and a reactor gas collection chamber; a gas outlet of the gas-solid separation unit I is connected to the reactor gas collection chamber; the reactor gas collection chamber is connected to a product gas delivery pipe; a inlet of the gas-solid separation unit II is connected to the riser reactor; a gas outlet of the gas-solid separation unit II is connected to the reactor gas collection chamber; a catalyst outlet of the gas-solid separation unit II is located above the open end of the inlet pipe of the reactor stripper and between the 1^{st} sub-distributor and the 2^{nd} sub-distributor.

Preferably, the reactor gas collection chamber is located at the inner top of the reactor shell.

Preferably, the gas-solid separation unit I employs one or more sets of gas-solid cyclone separators, each set comprising a first-stage gas-solid cyclone separator and a second-stage gas-solid cyclone separator.

Preferably, the fluidized bed regenerator is connected to the fluidized bed reactor and is configured to introduce a regeneration gas to regenerate the spent catalyst from the fluidized bed reactor, thereby obtaining a regenerated catalyst.

Preferably, the fluidized bed reactor is sequentially connected to the fluidized bed regenerator through a reactor stripper, a spent catalyst slide valve and a spent catalyst delivery pipe; wherein the inlet of the reactor stripper extends into the reactor shell of the fluidized bed reactor, with its open end located below the catalyst outlet of the gas-solid separation unit I and above the 1^{st} sub-distributor.

Preferably, the fluidized bed regenerator comprises a regenerator shell, wherein the shell enclosed by the regenerator shell is divided from top to bottom into a second gas-solid separation zone and a regeneration zone; the second gas-solid separation zone is provided with a regenerator gas-solid separation unit and a regenerator gas collection chamber; the regenerator gas collection chamber is located at the inner top of the regenerator shell and is provided with a flue gas delivery pipe; the gas outlet of the regenerator gas-solid separation unit is connected to the regenerator gas collection chamber; the lower section of the regeneration zone is provided with a regenerator distributor for introducing the regeneration gas.

Preferably, the regenerator gas-solid separation unit employs one or more sets of gas-solid cyclone separators, each set comprising a first-stage gas-solid cyclone separator and a second-stage gas-solid cyclone separator.

Preferably, the riser reactor is configured to introduce the riser reactor feedstock and the catalyst to react and produce aromatics, and a flow containing unreacted the riser reactor feedstock, aromatics and the catalyst enters the fluidized bed reactor through the outlet of the riser reactor.

Preferably, the inlet of the riser reactor is connected to the fluidized bed regenerator, and the catalyst introduced into the riser reactor is the regenerated catalyst produced in the fluidized bed regenerator.

Preferably, the fluidized bed regenerator is sequentially connected to the inlet of the riser reactor through a regenerator stripper, a regenerated catalyst slide valve and a pipeline.

Preferably, the inlet of the regenerator stripper extends into the regenerator shell of the fluidized bed regenerator and is located above the regenerator distributor.

According to another aspect of the present application, an its application method of the aforementioned device is provided, comprising: using the circulating fluidized bed reaction-regeneration device and a metal molecular sieve bifunctional catalyst to prepare aromatics.

Preferably, the method comprises: introducing naphtha feedstock into the reaction zone of the fluidized bed reactor through the 1^{st} sub-distributor of the reactor distributor, contacting with the catalyst from the riser reactor to generate a product gas flow containing BTX, light olefins, hydrogen, light alkanes, combustible gas, heavy aromatics and unconverted naphtha;
introducing methanol feedstock into the reaction zone of the fluidized bed reactor through the 2^{nd} to n^{th} sub-distributors of the reactor distributor respectively, undergoing methylation reaction with benzene and toluene in the product gas flow to generate para-xylene;
outputting the product gas from the fluidized bed reactor to downstream sections.

Preferably, the metal molecular sieve bifunctional catalyst employs a metal-modified HZSM-5 zeolite molecular sieve;
a metal used for a metal modification is at least one selected from the group consisting of La, Zn, Ga, Fe, Mo, and Cr;
the metal modification comprises: placing an HZSM-5 zeolite molecular sieve in a metal salt solution, and carrying out an impregnation, a drying and a calcination to obtain the metal-modified HZSM-5 zeolite molecular sieve.

Preferably, before outputting the product gas, the fluidized bed reactor first uses the gas-solid separation unit I for gas-solid separation to remove the spent catalyst entrained in the product gas flow.

Preferably, after entering the fluidized bed reactor, the catalyst from the riser reactor first undergoes gas-solid separation through the gas-solid separation unit II, and the catalyst with gas removed enters between the 1^{st} sub-distributor and the 2^{nd} sub-distributor through the catalyst outlet of the gas-solid separation unit II.

Preferably, the light olefins refer to ethylene and propylene;
the light alkanes refer to ethane and propane;
the combustible gas comprises methane and CO;
the heavy aromatics refer to aromatic hydrocarbons with nine or more carbon atoms per molecule.

Preferably, the naphtha feedstock is at least one selected from the group consisting of coal direct liquefaction naphtha, coal indirect liquefaction naphtha, straight-run naphtha and hydrocracked naphtha.

Preferably, the naphtha feedstock further comprises unconverted naphtha separated from the product gas flow, with the main components of the unconverted naphtha being linear aliphatic hydrocarbons, branched aliphatic hydrocarbons and naphthenes of C₄-C₁₂.

Preferably, the process conditions for the reaction zone of the fluidized bed reactor are: gas superficial velocity of 0.5-2.0 m/s, reaction temperature of 500-600°C, reaction pressure of 100-500 kPa, bed density of 150-700 kg/m³.

Optionally, the gas superficial velocity for the reaction zone of the fluidized bed reactor is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature for the reaction zone of the fluidized bed reactor is independently selected from any value among 500°C, 510°C, 520°C, 530°C, 540°C, 550°C, 560°C, 570°C, 580°C, 590°C, 600°C or any range between two values.

Optionally, the reaction pressure for the reaction zone of the fluidized bed reactor is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density for the reaction zone of the fluidized bed reactor is independently selected from any value among 150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Preferably, the method further comprises: introducing the spent catalyst generated in the fluidized bed reactor into the fluidized bed regenerator, introducing the regeneration gas into the regeneration zone of the fluidized bed regenerator to contact with the spent catalyst and react to produce the flue gas and the regenerated catalyst.

Preferably, the flue gas enters the regenerator gas-solid separation unit to remove regenerated catalyst entrained in it, then enters the regenerator gas collection chamber and is delivered to downstream sections through the flue gas delivery pipe.

Preferably, the method further comprises: the regenerated catalyst sequentially passes through the regenerator stripper and the regenerated catalyst slide valve to enter the riser reactor.

Preferably, the carbon content in the spent catalyst is in a range from 1.0 wt% to 3.0 wt%.

Preferably, the carbon content in the regenerated catalyst is ≤0.5 wt%.

Preferably, the regeneration gas is at least one selected from the group consisting of oxygen, air and oxygen-enriched air.

Preferably, the process conditions for the regeneration zone of the fluidized bed regenerator are: gas superficial velocity of 0.5-2.0 m/s, regeneration temperature of 600-750°C, regeneration pressure of 100-500 kPa, bed density of 150-700 kg/m³.

Optionally, the gas superficial velocity for the regeneration zone of the fluidized bed regenerator is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the regeneration temperature for the regeneration zone of the fluidized bed regenerator is independently selected from any value among 600°C, 615°C, 630°C, 645°C, 670°C, 685°C, 700°C, 715°C, 730°C, 745°C, 750°C or any range between two values.

Optionally, the regeneration pressure for the regeneration zone of the fluidized bed regenerator is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density for the regeneration zone of the fluidized bed regenerator is independently selected from any value among150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Preferably, the method further comprises: introducing the riser reactor feedstock and the catalyst into the riser reactor to react and produce aromatics;
the flow containing the unreacted riser reactor feedstock, aromatics and the catalyst enters the gas-solid separation unit II of the fluidized bed reactor from the outlet of the riser reactor.

Preferably, the catalyst is the regenerated catalyst from the fluidized bed regenerator.

Preferably, the riser reactor feedstock comprises water vapor and light alkanes separated from the product gas flow.

Preferably, the water vapor content in the riser reactor feedstock is in a range from 0 wt% to 50 wt%.

Preferably, the process conditions for the riser reactor are: gas superficial velocity of 3.0-10.0 m/s, temperature of 580-700°C, pressure of 100-500 kPa, bed density of 50-150 kg/m³.

Optionally, the gas superficial velocity for the riser reactor is independently selected from any value among 3.0m/s, 3.5m/s, 4.0m/s, 4.5m/s, 5.0m/s, 5.5m/s, 6.0m/s, 6.5m/s, 7.0m/s, 7.5m/s, 8.0m/s, 8.5m/s, 9.0m/s, 9.5m/s, 10.0m/s or any range between two values.

Optionally, the temperature for the riser reactor is independently selected from any value among 580°C, 590°C, 600°C, 610°C, 620°C, 630°C, 640°C, 650°C, 660°C, 670°C, 680°C, 690°C, 700°C or any range between two values.

Optionally, the pressure for the riser reactor is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density for the riser reactor is independently selected from any value among 50kg/m³, 60kg/m³, 70kg/m³, 80kg/m³, 90kg/m³, 100kg/m³, 110kg/m³, 120kg/m³, 130kg/m³, 140kg/m³, 150kg/m³ or any range between two values.

The naphtha feedstock has an aromatic potential of 0-80 wt%, with a single-pass conversion rate of 60-80 wt% for naphtha and approximately 100 wt% for methanol. The unconverted naphtha separated from the product gas is recycled as feedstock to the fluidized bed reactor, while a portion of the light alkanes separated from the product gas is returned as feedstock to the riser reactor. The final product distribution is as follows: 60-71 wt% BTX, 9-16 wt% light olefins, 3-7 wt% hydrogen, 2-7 wt% light alkanes, 4-6 wt% combustible gas, 3-7 wt% heavy aromatics, and 0.5-1 wt% coke. The para-xylene content in the mixed xylenes is 60-75 wt%.

The beneficial effects of the present application include:
1) The present invention can efficiently and selectively convert linear and branched aliphatic hydrocarbons into aromatics, and has a wide range of raw material adaptability, and can use naphtha with low aromatic potential content as a raw material to prepare aromatics.
2) The present application realizes the aromatization of light alkanes through the riser reactor and a metal molecular sieve bifunctional catalyst, greatly improving the aromatics yield of naphtha-to-aromatics technology.
3) The circulating fluidized bed reaction-regeneration device in the present application is equipped with a fluidized bed reactor, which includes multiple sub-distributors and a gas-solid separation unit II. Naphtha enters the reaction zone via the 1^{st} sub-distributor, while methanol enters the reaction zone separately through the 2^{nd} to n^{th} sub-distributors. The gas-solid separation unit II directly delivers the higher-temperature catalyst from the riser reactor above the 1^{st} sub-distributor. The fluidized bed reactor is suitable for controlling cascade reactions. Naphtha is first converted into benzene and toluene in the lower part of the reaction zone, then flows upward to the middle and upper parts of the reaction zone, where benzene, toluene and methanol undergo methylation reactions to further produce para-xylene, thereby increasing para-xylene yield. The higher-temperature catalyst from the riser reactor directly enters the lower part of the reaction zone, which helps provide the heat required for the conversion of naphtha into aromatics and improves naphtha conversion rate. Methanol directly enters the middle and upper parts of the reaction zone, effectively reducing the residence time of para-xylene in the reaction zone, inhibiting the isomerization reaction of para-xylene, increasing the para-xylene content in xylenes (up to 75wt% under optimal process conditions), while significantly reducing the separation energy consumption of p-xylene. In short, in the fluidized bed reactor, the naphtha feedstock flows from bottom to top. During its conversion into aromatics, by stepwise addition of methylation feedstock (methanol), the process of cascade reactions (naphtha-benzene / toluene-p-xylene) is controlled, achieving increased p-xylene production.
4) In the present application, the naphtha aromatization reaction is strongly endothermic, requiring absorption of 1.1-1.6MJ heat per kg of naphtha converted to aromatics. The methylation reaction of methanol and aromatics is strongly exothermic, releasing over 2.0MJ heat per kg of methanol converted to methyl groups on aromatics. Therefore, using benzene, toluene and methanol to produce para-xylene can provide heat in situ for the coupled naphtha and methanol aromatics production reaction, achieving autothermal balance.
5) The circulating fluidized bed reaction-regeneration device of the present application is equipped with an independent riser reactor. Light alkanes are very stable and require high reaction temperatures. Therefore, in the circulating fluidized bed reaction-regeneration device of this application, the high-temperature regenerated catalyst first enters the riser reactor and contacts light alkanes. The light alkanes undergo aromatization reaction under the action of the catalyst, improving reaction rate and aromatic yield. By connecting the high-temperature riser reactor and the relatively lower-temperature fluidized bed reactor in series, the circulating fluidized bed reaction-regeneration device of this application achieves the beneficial effects of reducing light alkane yield and increasing aromatic yield.

### BRIEF DESCRIPTION OF THE DRAWINGS

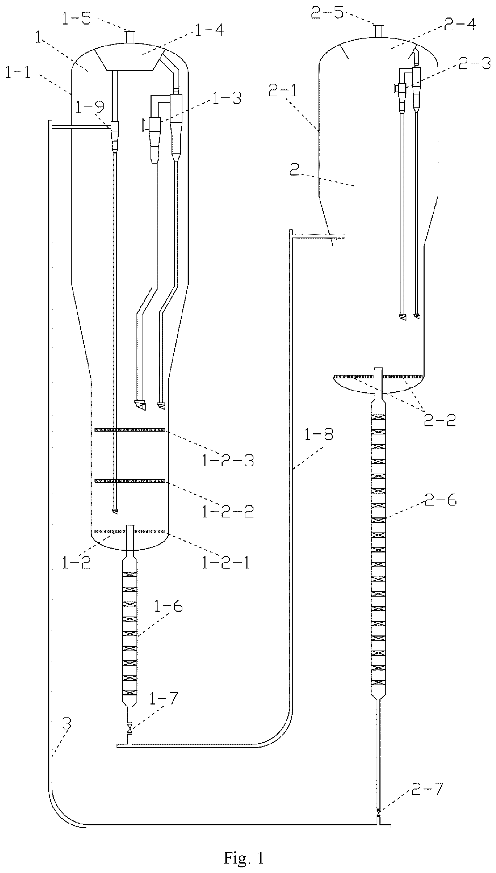

Fig. 1 is a schematic diagram of a circulating fluidized bed reaction-regeneration device according to one embodiment of the present application.

List of Components and Reference Numerals:
1: fluidized bed reactor; 1-1: reactor shell; 1-2: reactor distributor;
1-3: gas-solid separation unit I; 1-4: reactor gas collection chamber I; 1-5: product gas delivery pipe;
1-6: reactor stripper; 1-7: spent catalyst slide valve; 1-8: spent catalyst delivery pipe;
1-9: gas-solid separation unit II;
1-2-1: 1^{st} sub-distributor; 1-2-2: 2^{nd} sub-distributor; 1-2-3: 3^{rd} sub-distributor;
2: fluidized bed regenerator; 2-1: regenerator shell; 2-2: regenerator distributor;
2-3: regenerator gas-solid separation unit; 2-4: regenerator gas collection chamber; 2-5: flue gas delivery pipe;
2-6: regenerator stripper; 2-7: regenerated catalyst slide valve;
3: riser reactor.

### DETAILED DESCRIPTION OF THE EMBODIMENTS

The present application will be described in detail below with reference to examples, but the present application is not limited to these examples.

The present application provides a circulating fluidized bed reaction-regeneration device, comprising: a fluidized bed reactor, the fluidized bed reactor being used for introducing naphtha feedstock and methanol feedstock, the naphtha feedstock contacting with the catalyst from the riser reactor to react and produce a product gas flow containing BTX and the spent catalyst, the methanol feedstock undergoing methylation reaction with benzene and toluene in the said product gas stream containing BTX to generate para-xylene; performing the gas-solid separation on the product gas flow, with the separated product gas being delivered to downstream sections, the unconverted naphtha being recycled as feedstock to the fluidized bed reactor, a portion of light alkanes being recycled as feedstock to the riser reactor, and the spent catalyst being introduced into the fluidized bed regenerator;
the inlet of the riser reactor being connected to the fluidized bed regenerator, and the outlet of the riser reactor being connected to the fluidized bed reactor.

Please refer to Fig. 1. The device comprises a fluidized bed reactor 1, a fluidized bed regenerator 2 and a riser reactor 3.

The fluidized bed reactor 1 comprises: a reactor shell 1-1, a reactor distributor 1-2, a gas-solid separation unit I 1-3, a reactor gas collection chamber 1-4, a product gas delivery pipe 1-5, a reactor stripper 1-6, a spent catalyst slide valve 1-7, a spent catalyst delivery pipe 1-8, and a gas-solid separation unit II 1-9.

The reactor distributor 1-2 comprises: a 1^{st} sub-distributor 1-2-1, a 2^{nd} sub-distributor 1-2-2, and a 3^{rd} sub-distributor 1-2-3.

The reactor shell 1-1 comprises an upper reactor shell and a lower reactor shell. The upper reactor shell encloses a gas-solid separation zone, and the lower reactor shell encloses a reaction zone. The reactor shell 1-1 is provided with an outlet of the riser reactor 3.

The reaction zone is provided with the reactor distributor 1-2. The reactor distributor comprises three sub-distributors, which are sequentially arranged from bottom to top as the 1^{st} sub-distributor 1-2-1 to the 3^{rd} sub-distributor 1-2-3. The 1^{st} sub-distributor 1-2-1 is used for introducing naphtha feedstock. The 2^{nd} sub-distributor 1-2-2 to the 3^{rd} sub-distributor 1-2-3 are used for introducing methanol feedstock.

The reactor shell 1-1 is further provided with the gas-solid separation unit I 1-3, the gas-solid separation unit II 1-9 and the reactor gas collection chamber 1-4. The reactor gas collection chamber 1-4 is located at the inner top of the reactor shell. The gas outlet of the gas-solid separation unit I 1-3 is connected to the reactor gas collection chamber 1-4. The reactor gas collection chamber 1-4 is connected to the product gas delivery pipe 1-5. The catalyst outlet end of the gas-solid separation unit I 1-3 is located above the opening end of the inlet pipe of the reactor stripper 1-6. The inlet of the gas-solid separation unit II 1-9 is connected to the riser reactor 3. The gas outlet of the gas-solid separation unit II 1-9 is connected to the reactor gas collection chamber 1-4. The catalyst outlet end of the gas-solid separation unit II 1-9 is located above the opening end of the inlet pipe of the reactor stripper 1-6, and between the 1^{st} sub-distributor 1-2-1 and the 2nd sub-distributor 1-2-2.

The reactor stripper 1-6 is provided beneath the reaction zone. The inlet of the reactor stripper 1-6 is located inside the reactor shell 1-1. The outlet of the reactor stripper 1-6 is located outside the reactor shell 1-1 and is connected to the spent catalyst slide valve 1-7. The opening end of the inlet of the reactor stripper 1-6 is located above the 1^{st} sub-distributor.

The spent catalyst slide valve 1-7 is provided beneath the reactor stripper 1-6. The inlet of the spent catalyst slide valve 1-7 is connected to the outlet of the reactor stripper 1-6. The outlet of the spent catalyst slide valve 1-7 is connected to the inlet of the spent catalyst delivery pipe 1-8. The outlet of the spent catalyst delivery pipe 1-8 is connected to the regenerator shell 2-1.

The spent catalyst slide valve 1-7 is used for controlling the circulation amount of spent catalyst.

In a preferred embodiment, the gas-solid separation unit I 1-3 employs one or more sets of gas-solid cyclone separators, each set comprising a first-stage gas-solid cyclone separator and a second-stage gas-solid cyclone separator.

The fluidized bed regenerator 2 comprises: a regenerator shell 2-1, a regenerator distributor 2-2, a regenerator gas-solid separation unit 2-3, a regenerator gas collection chamber 2-4, a flue gas delivery pipe 2-5, a regenerator stripper 2-6, and a regenerated catalyst slide valve 2-7.

The regenerator shell 2-1 comprises an upper regenerator shell and a lower regenerator shell. The upper regenerator shell encloses a gas-solid separation zone, and the lower regenerator shell encloses a regeneration zone. The regenerator shell 2-1 is provided with an outlet of the spent catalyst delivery pipe 1-8.

The regenerator distributor 2-2 is provided at lower part of the regenerator in the regeneration zone. The regenerator distributor 2-2 is used for introducing the regeneration gas.

The regenerator shell 2-1 is further provided with the regenerator gas-solid separation unit 2-3 and the regenerator gas collection chamber 2-4. The regenerator gas collection chamber 2-4 is located at the inner top of the regenerator shell 2-1. The gas outlet of the regenerator gas-solid separation unit 2-3 is connected to the regenerator gas collection chamber 2-4. The regenerator gas collection chamber 2-4 is connected to the flue gas delivery pipe 2-5. The catalyst outlet end of the regenerator gas-solid separation unit 2-3 is located above the opening end of the inlet pipe of the regenerator stripper 2-6.

The regenerator stripper 2-6 is provided beneath the regeneration zone. The inlet of the regenerator stripper 2-6 is located inside the regenerator shell 2-1. The outlet of the regenerator stripper 2-6 is located outside the regenerator shell 2-1 and is connected to the regenerated catalyst slide valve 2-7. The opening end of the inlet of the regenerator stripper 2-6 is located above the regenerator distributor 2-2.

The regenerated catalyst slide valve 2-7 is provided beneath the regenerator stripper 2-6. The inlet of the regenerated catalyst slide valve 2-7 is connected to the outlet of the regenerator stripper 2-6.

The regenerated catalyst slide valve 2-7 is used for controlling the circulation amount of regenerated catalyst.

In a preferred embodiment, the regenerator gas-solid separation unit 2-3 employs one or more sets of gas-solid cyclone separators, each set comprising a first-stage gas-solid cyclone separator and a second-stage gas-solid cyclone separator.

The inlet of the riser reactor 3 is connected to the regenerated catalyst slide valve 2-7. The outlet of the riser reactor 3 is connected to the inlet of the gas-solid separation unit II 1-9.

The present application also provides an its application method of the aforementioned device, especially a method for coupling naphtha and methanol to produce aromatics, comprising: using the circulating fluidized bed reaction-regeneration device and a metal molecular sieve bifunctional catalyst to prepare aromatics.

The metal molecular sieve bifunctional catalysts in Examples 1 to 5 employ a metal-modified HZSM-5 zeolite molecular sieve. The metal used for a metal modification is at least one selected from the group consisting of La, Zn, Ga, Fe, Mo, and Cr; the metal modification comprises: placing an HZSM-5 zeolite molecular sieve in a metal salt solution, and carrying out an impregnation, a drying and a calcination to obtain the metal-modified HZSM-5 zeolite molecular sieve.

In a preferred embodiment, the method comprises the following steps:
a) naphtha enters the reaction zone of the fluidized bed reactor 1 through the 1^{st} sub-distributor 1-2-1 of the reactor distributor 1-2, and contacts with catalyst from the riser reactor 3 to generate a product gas flow containing BTX, light olefins, hydrogen, light alkanes, combustible gas, heavy aromatics and unconverted naphtha; the catalyst from the riser reactor 3 enters the gas-solid separation unit II 1-9 to achieve gas-solid separation, then the degassed catalyst enters between the 1^{st} sub-distributor 1-2-1 and the 2^{nd} sub-distributor 1-2-2; methanol enters the reaction zone of the fluidized bed reactor 1 through the 2^{nd} sub-distributor 1-2-2 to the 3^{rd} sub-distributor 1-2-3 of the reactor distributor 1-2 respectively, and undergoes methylation reaction with benzene and toluene in the product gas flow to generate para-xylene; the catalyst from the riser reactor 3 is converted into spent catalyst through coking in the fluidized bed reactor 1; the product gas flow enters the gas-solid separation unit I 1-3 to remove the entrained spent catalyst, then enters the gas collection chamber 1-4, and is delivered to downstream units through the product gas delivery pipe 1-5; the spent catalyst in the reaction zone enters the stripper 1-6 through the opening end of the inlet pipe of the stripper 1-6 for stripping, and after stripping, passes through the spent catalyst slide valve 1-7 and the spent catalyst delivery pipe 1-8 to enter the fluidized bed regenerator 2.
b) the regeneration gas is introduced into the regeneration zone of the fluidized bed regenerator 2 through the regenerator distributor 2-2, and contacts with the spent catalyst; the coke on the spent catalyst reacts with the regeneration gas to generate flue gas, while the spent catalyst is converted into regenerated catalyst; the flue gas enters the regenerator gas-solid separation unit 2-3 to remove the entrained regenerated catalyst, then enters the regenerator gas collection chamber 2-4, and is delivered to downstream units through the flue gas delivery pipe 2-5; the regenerated catalyst sequentially passes through the regenerator stripper 2-6 and the regenerated catalyst slide valve 2-7 to enter the riser reactor 3.
c) The riser reactor feedstock is introduced into the riser reactor 3, contacts and reacts with the regenerated catalyst from the fluidized bed regenerator 2; the riser reactor feedstock is converted into aromatics under the action of the catalyst, then the flow containing unreacted riser reactor feedstock, aromatics and catalyst enters the gas-solid separation unit II 1-9 in the fluidized bed reactor 1 from the outlet of the riser reactor 3.

The light olefins refer to ethylene and propylene.

The light alkanes refer to ethane and propane.

The combustible gas comprises methane and CO.

The heavy aromatics refer to aromatic hydrocarbons with nine or more carbon atoms per molecule.

In a preferred embodiment, the naphtha feedstock is at least one selected from the group consisting of coal direct liquefaction naphtha, coal indirect liquefaction naphtha, straight-run naphtha and hydrocracked naphtha.

In a preferred embodiment, the naphtha feedstock further comprises unconverted naphtha separated from the product gas flow.

In a preferred embodiment, the carbon content in the spent catalyst is in a range from 1.0 wt% to 3.0 wt%.

In a preferred embodiment, the process conditions for the reaction zone of the fluidized bed reactor are: gas superficial velocity of 0.5-2.0 m/s, reaction temperature of 500-600°C, reaction pressure of 100-500 kPa, bed density of 150-700 kg/m³.

Optionally, the gas superficial velocity for the reaction zone of the fluidized bed reactor is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature for the reaction zone of the fluidized bed reactor is independently selected from any value among 500°C, 510°C, 520°C, 530°C, 540°C, 550°C, 560°C, 570°C, 580°C, 590°C, 600°C or any range between two values.

Optionally, the reaction pressure for the reaction zone of the fluidized bed reactor is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density for the reaction zone of the fluidized bed reactor is independently selected from any value among 150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

In a preferred embodiment, the carbon content in the regenerated catalyst is ≤0.5 wt%.

In a preferred embodiment, the regeneration gas is at least one selected from the group consisting of oxygen, air and oxygen-enriched air.

In a preferred embodiment, the process conditions for the regeneration zone of the fluidized bed regenerator are: gas superficial velocity of 0.5-2.0 m/s, regeneration temperature of 600-750°C, regeneration pressure of 100-500 kPa, bed density of 150-700 kg/m³.

Optionally, the gas superficial velocity for the regeneration zone of the fluidized bed regenerator is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the regeneration temperature for the regeneration zone of the fluidized bed regenerator is independently selected from any value among 600°C, 615°C, 630°C, 645°C, 670°C, 685°C, 700°C, 715°C, 730°C, 745°C, 750°C or any range between two values.

Optionally, the regeneration pressure for the regeneration zone of the fluidized bed regenerator is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density for the regeneration zone of the fluidized bed regenerator is independently selected from any value among150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

In a preferred embodiment, the riser reactor feedstock comprises water vapor and light alkanes separated from the product gas flow.

In a preferred embodiment, the water vapor content in the riser reactor feedstock is in a range from 0 wt% to 50 wt%.

In a preferred embodiment, the process conditions for the riser reactor are: gas superficial velocity of 3.0-10.0 m/s, temperature of 580-700°C, pressure of 100-500 kPa, bed density of 50-150 kg/m³.

Optionally, the gas superficial velocity for the riser reactor is independently selected from any value among 3.0m/s, 3.5m/s, 4.0m/s, 4.5m/s, 5.0m/s, 5.5m/s, 6.0m/s, 6.5m/s, 7.0m/s, 7.5m/s, 8.0m/s, 8.5m/s, 9.0m/s, 9.5m/s, 10.0m/s or any range between two values.

Optionally, the temperature for the riser reactor is independently selected from any value among 580°C, 590°C, 600°C, 610°C, 620°C, 630°C, 640°C, 650°C, 660°C, 670°C, 680°C, 690°C, 700°C or any range between two values.

Optionally, the pressure for the riser reactor is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density for the riser reactor is independently selected from any value among 50kg/m³, 60kg/m³, 70kg/m³, 80kg/m³, 90kg/m³, 100kg/m³, 110kg/m³, 120kg/m³, 130kg/m³, 140kg/m³, 150kg/m³ or any range between two values.

The naphtha feedstock has an aromatic potential of 0-80 wt%, with a single-pass conversion rate of 60-80 wt% for naphtha and approximately 100 wt% for methanol. The unconverted naphtha separated from the product gas is recycled as feedstock to the fluidized bed reactor, while a portion of the light alkanes separated from the product gas is returned as feedstock to the riser reactor. The final product distribution is as follows: 60-71 wt% BTX, 9-16 wt% light olefins, 3-7 wt% hydrogen, 2-7 wt% light alkanes, 4-6 wt% combustible gas, 3-7 wt% heavy aromatics, and 0.5-1 wt% coke. The para-xylene content in the mixed xylenes is 60-75 wt%.

### Example 1

This embodiment employs the device illustrated in Fig. 1.

Naphtha feedstock fed to the fluidized bed reactor is coal direct liquefaction naphtha with an aromatics potential content of 78 wt%, including unconverted naphtha recycled from the separated product gas flow.

The fluidized bed reaction zone conditions: gas superficial velocity is 0.5 m/s, reaction temperature is 600°C, reaction pressure is 100 kPa, bed density is 700 kg/m³.

Regeneration gas is air.

Regeneration zone conditions in the fluidized bed regenerator: gas superficial velocity is 0.5 m/s, regeneration temperature is 745°C, regeneration pressure is 100 kPa, bed density is 700 kg/m³.

Riser reactor feedstock is light alkanes separated from the product gas flow.

Riser reactor conditions: gas superficial velocity is 3.0 m/s, temperature is 690°C, pressure is 100 kPa, bed density is 150 kg/m³.

Carbon content in the spend catalyst is 1.0 wt%, and carbon content in the regenerated catalyst is 0.1 wt%.

Single-pass conversion of naphtha feedstock into the n fluidized bed reactor is 60 wt%.

Product distribution: 71 wt% BTX, 9 wt% light olefins, 4 wt% hydrogen, 2 wt% light alkanes, 6 wt% combustible gas, 7 wt% heavy aromatics, 1 wt% coke. The content of p-xylene in the mixed xylene in the product is 66 wt%.

### Example 2

This embodiment employs the device illustrated in Fig. 1.

Naphtha feedstock fed to the fluidized bed reactor is coal direct liquefaction naphtha with an aromatics potential content of 0.1 wt%, including unconverted naphtha recycled from the separated product gas flow.

Fluidized bed reaction zone conditions: gas superficial velocity is 2.0 m/s, reaction temperature is 510°C, reaction pressure is 500 kPa, bed density is 150 kg/m³.

Regeneration gas is oxygen.

Regeneration zone conditions in the fluidized bed regenerator: gas superficial velocity is 2.0 m/s, regeneration temperature is 610°C, regeneration pressure is 500 kPa, bed density is 150 kg/m³.

Riser reactor feedstock is light alkanes separated from the product gas flow and water vapor, with a water vapor content of 50 wt%.

Riser reactor conditions: gas superficial velocity is 10.0 m/s, temperature is 580°C, pressure is 500 kPa, bed density is 50 kg/m³.

Carbon content in the spend catalyst is 3.0 wt%, and carbon content in the regenerated catalyst is 0.3 wt%.

Single-pass conversion of naphtha feedstock into the fluidized bed reactor is 77 wt%.

Product distribution: 62 wt% BTX, 15 wt% light olefins, 7 wt% hydrogen, 4 wt% light alkanes, 6 wt% combustible gas, 5.5 wt% heavy aromatics, 0.5 wt% coke. The content of p-xylene in the mixed xylene in the product is 70 wt%.

### Example 3

This embodiment employs the device illustrated in Fig. 1.

Naphtha feedstock fed to the fluidized bed reactor is coal direct liquefaction naphtha with an aromatics potential content of 3 wt%, including unconverted naphtha recycled from the separated product gas flow.

Fluidized bed reaction zone conditions: gas superficial velocity is 1.2 m/s, reaction temperature is 550°C, reaction pressure is 120 kPa, bed density is 260 kg/m³.

Regeneration gas is oxygen-enriched air.

Regeneration zone conditions in the fluidized bed regenerator: gas superficial velocity is 1.2 m/s, regeneration temperature is 650°C, regeneration pressure is 120 kPa, bed density is 260 kg/m³.

Riser reactor feedstock is light alkanes separated from the product gas flow and water vapor, with a water vapor content of 25 wt%.

Riser reactor conditions: gas superficial velocity is 7.0 m/s, temperature is 630°C, pressure is 120 kPa, bed density is 80 kg/m³.

Carbon content in the spend catalyst is 2.4 wt%, and carbon content in the regenerated catalyst is 0.2 wt%.

Single-pass conversion of naphtha feedstock into the fluidized bed reactor is 80 wt%.

Product distribution: 61 wt% BTX, 16 wt% light olefins, 7 wt% hydrogen, 7 wt% light alkanes, 5 wt% combustible gas, 3 wt% heavy aromatics, 1 wt% coke. The content of p-xylene in the mixed xylene in the product is 60 wt%.

### Example 4

This embodiment employs the device illustrated in Fig. 1.

Naphtha feedstock fed to the fluidized bed reactor is coal direct liquefaction naphtha with an aromatics potential content of 46 wt%, including unconverted naphtha recycled from the separated product gas flow.

Fluidized bed reaction zone conditions: gas superficial velocity is 1.8 m/s, reaction temperature is 590°C, reaction pressure is 200 kPa, bed density is 220 kg/m³.

Regeneration gas is air.

Regeneration zone conditions in the fluidized bed regenerator: gas superficial velocity is 1.8 m/s, regeneration temperature is 700°C, regeneration pressure is 200 kPa, bed density is 220 kg/m³.

Riser reactor feedstock is light alkanes separated from the product gas flow and water vapor, with a water vapor content of 50 wt%.

Riser reactor conditions: gas superficial velocity is 5.0 m/s, temperature is 660°C, pressure is 220 kPa, bed density is 110 kg/m³.

Carbon content in the spend catalyst is 1.8 wt%, and carbon content in the regenerated catalyst is 0.1 wt%.

Single-pass conversion of naphtha feedstock into the fluidized bed reactor is 66 wt%.

Product distribution: 66 wt% BTX, 14 wt% light olefins, 3 wt% hydrogen, 6 wt% light alkanes, 4 wt% combustible gas, 6.4 wt% heavy aromatics, 0.6 wt% coke. The content of p-xylene in the mixed xylene in the product is 75 wt%.

### Example 5

This embodiment employs the device illustrated in Fig. 1.

Naphtha feedstock fed to the fluidized bed reactor is coal direct liquefaction naphtha with an aromatics potential content of 64 wt%, including unconverted naphtha recycled from the separated product gas flow.

Fluidized bed reaction zone conditions: gas superficial velocity is 1.0 m/s, reaction temperature is 580°C, reaction pressure is 150 kPa, bed density is 350 kg/m³.

Regeneration gas is air.

Regeneration zone conditions in the fluidized bed regenerator: gas superficial velocity is 1.0 m/s, regeneration temperature is 680°C, regeneration pressure is 150 kPa, bed density is 350 kg/m³.

Riser reactor feedstock is light alkanes separated from the product gas flow and water vapor, with a water vapor content of 40 wt%.

Riser reactor conditions: gas superficial velocity is 7.0 m/s, temperature is 650°C, pressure is 150 kPa, bed density is 80 kg/m³.

Carbon content in the spend catalyst is 1.4 wt%, and carbon content in the regenerated catalyst is 0.5 wt%.

Single-pass conversion of naphtha feedstock into the fluidized bed reactor is 71 wt%.

Product distribution: 70 wt% BTX, 11.3 wt% light olefins, 5 wt% hydrogen, 2 wt% light alkanes, 5 wt% combustible gas, 6 wt% heavy aromatics, 0.7 wt% coke. The content of p-xylene in the mixed xylene in the product is 71 wt%.

The above examples are merely few examples of the present application, and do not limit the present application in any form. Although the present application is disclosed as above with preferred examples, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

## Claims

1. A circulating fluidized bed reaction-regeneration device, **characterized in that** the device comprises a fluidized bed reactor, a fluidized bed regenerator and a riser reactor;
the fluidized bed reactor is configured to introduce naphtha feedstock and methanol feedstock, wherein the naphtha feedstock contacts with a catalyst from the riser reactor to produce a product gas flow containing BTX and a spent catalyst, and the methanol feedstock undergoes methylation reaction with benzene and toluene in the product gas flow containing BTX to generate para-xylene; the product gas flow undergoes gas-solid separation, with the separated product gas being delivered to downstream sections, unconverted naphtha being recycled as feedstock to the fluidized bed reactor, and partial light alkanes being recycled as feedstock to the riser reactor, while the spent catalyst is introduced into the fluidized bed regenerator;
the inlet of the riser reactor is connected to the fluidized bed regenerator, and the outlet of the riser reactor is connected to the fluidized bed reactor.

2. The circulating fluidized bed reaction-regeneration device according to claim 1, **characterized in that** the fluidized bed reactor comprises a reactor shell, wherein the region enclosed by the reactor shell is divided from top to bottom into a first gas-solid separation zone and a reaction zone, the reaction zone being provided with a reactor distributor comprising n sub-distributors arranged sequentially from bottom to top as the 1^{st} sub-distributor to the n^{th} sub-distributor, where n≥2 and n≤10; the 1^{st} sub-distributor is configured to introduce naphtha feedstock; and the 2^{nd} to n^{th} sub-distributors are configured to introduce methanol feedstock.

3. The circulating fluidized bed reaction-regeneration device according to claim 2, **characterized in that** the first gas-solid separation zone is provided with a gas-solid separation unit I, a gas-solid separation unit II and a reactor gas collection chamber; a gas outlet of the gas-solid separation unit I is connected to the reactor gas collection chamber; the reactor gas collection chamber is connected to a product gas delivery pipe; a inlet of the gas-solid separation unit II is connected to the riser reactor; a gas outlet of the gas-solid separation unit II is connected to the reactor gas collection chamber; a catalyst outlet of the gas-solid separation unit II is located above the open end of the inlet pipe of the reactor stripper and between the 1^{st} sub-distributor and the 2^{nd} sub-distributor.

4. The circulating fluidized bed reaction-regeneration device according to claim 3, **characterized in that** the reactor gas collection chamber is located at the inner top of the reactor shell.

5. The circulating fluidized bed reaction-regeneration device according to claim 3, **characterized in that** the gas-solid separation unit I employs one or more sets of gas-solid cyclone separators, each set comprising a first-stage gas-solid cyclone separator and a second-stage gas-solid cyclone separator.

6. The circulating fluidized bed reaction-regeneration device according to claim 1, **characterized in that** the fluidized bed regenerator is connected to the fluidized bed reactor and is configured to introduce a regeneration gas to regenerate the spent catalyst from the fluidized bed reactor, thereby obtaining a regenerated catalyst.

7. The circulating fluidized bed reaction-regeneration device according to claim 6, **characterized in that** the fluidized bed reactor is sequentially connected to the fluidized bed regenerator through a reactor stripper, a spent catalyst slide valve and a spent catalyst delivery pipe; wherein the inlet of the reactor stripper extends into the reactor shell of the fluidized bed reactor, with its open end located below the catalyst outlet of the gas-solid separation unit I and above the 1^{st} sub-distributor.

8. The circulating fluidized bed reaction-regeneration device according to claim 1, **characterized in that** the fluidized bed regenerator comprises a regenerator shell, wherein the shell enclosed by the regenerator shell is divided from top to bottom into a second gas-solid separation zone and a regeneration zone; the second gas-solid separation zone is provided with a regenerator gas-solid separation unit and a regenerator gas collection chamber; the regenerator gas collection chamber is located at the inner top of the regenerator shell and is provided with a flue gas delivery pipe; the gas outlet of the regenerator gas-solid separation unit is connected to the regenerator gas collection chamber; the lower section of the regeneration zone is provided with a regenerator distributor for introducing the regeneration gas.

9. The circulating fluidized bed reaction-regeneration device according to claim 8, **characterized in that** the regenerator gas-solid separation unit employs one or more sets of gas-solid cyclone separators, each set comprising a first-stage gas-solid cyclone separator and a second-stage gas-solid cyclone separator.

10. The circulating fluidized bed reaction-regeneration device according to claim 1, **characterized in that** the riser reactor is configured to introduce the riser reactor feedstock and the catalyst to react and produce aromatics, and a flow containing unreacted the riser reactor feedstock, aromatics and the catalyst enters the fluidized bed reactor through the outlet of the riser reactor.

11. The circulating fluidized bed reaction-regeneration device according to claim 1, **characterized in that** the inlet of the riser reactor is connected to the fluidized bed regenerator, and the catalyst introduced into the riser reactor is the regenerated catalyst produced in the fluidized bed regenerator.

12. The circulating fluidized bed reaction-regeneration device according to claim 11, **characterized in that** the fluidized bed regenerator is sequentially connected to the inlet of the riser reactor through a regenerator stripper, a regenerated catalyst slide valve and a pipeline.

13. The circulating fluidized bed reaction-regeneration device according to claim 12, **characterized in that** the inlet of the regenerator stripper extends into the regenerator shell of the fluidized bed regenerator and is located above the regenerator distributor.

14. An use method based on the device according to any one of claims 1 to 13, **characterized in that** the method comprises: using the circulating fluidized bed reaction-regeneration device and a metal molecular sieve bifunctional catalyst to prepare aromatics.

15. The use method according to claim 14, **characterized in that** the metal molecular sieve bifunctional catalyst employs a metal-modified HZSM-5 zeolite molecular sieve;
a metal used for a metal modification is at least one selected from the group consisting of La, Zn, Ga, Fe, Mo, and Cr;
the metal modification comprises: placing an HZSM-5 zeolite molecular sieve in a metal salt solution, and carrying out an impregnation, a drying and a calcination to obtain the metal-modified HZSM-5 zeolite molecular sieve.

16. The use method according to claim 14, **characterized in that** the method comprises:
introducing naphtha feedstock into the reaction zone of the fluidized bed reactor through the 1^{st} sub-distributor of the reactor distributor, contacting with the catalyst from the riser reactor to generate a product gas flow containing BTX, light olefins, hydrogen, light alkanes, combustible gas, heavy aromatics and unconverted naphtha;
introducing methanol feedstock into the reaction zone of the fluidized bed reactor through the 2^{nd} to n^{th} sub-distributors of the reactor distributor respectively, undergoing methylation reaction with benzene and toluene in the product gas flow to generate para-xylene;
outputting the product gas from the fluidized bed reactor to downstream sections.

17. The use method according to claim 16, **characterized in that** before outputting the product gas, the fluidized bed reactor first uses the gas-solid separation unit I for gas-solid separation to remove the spent catalyst entrained in the product gas flow.

18. The use method according to claim 16, **characterized in that** after entering the fluidized bed reactor, the catalyst from the riser reactor first undergoes gas-solid separation through the gas-solid separation unit II, and the catalyst with gas removed enters between the 1^{st} sub-distributor and the 2^{nd} sub-distributor through the catalyst outlet of the gas-solid separation unit II.

19. The use method according to claim 16, **characterized in that** the light olefins refer to ethylene and propylene;
the light alkanes refer to ethane and propane;
the combustible gas comprises methane and CO;
the heavy aromatics refer to aromatic hydrocarbons with nine or more carbon atoms per molecule.

20. The use method according to claim 16, **characterized in that** the naphtha feedstock is at least one selected from the group consisting of coal direct liquefaction naphtha, coal indirect liquefaction naphtha, straight-run naphtha and hydrocracked naphtha.

21. The use method according to claim 20, **characterized in that** the naphtha feedstock further comprises unconverted naphtha separated from the product gas flow, with the main components of the unconverted naphtha being linear aliphatic hydrocarbons, branched aliphatic hydrocarbons and naphthenes of C₄-C₁₂.

22. The use method according to claim 16, **characterized in that** the process conditions for the reaction zone of the fluidized bed reactor are: gas superficial velocity of 0.5-2.0 m/s, reaction temperature of 500-600°C, reaction pressure of 100-500 kPa, bed density of 150-700 kg/m³.

23. The use method according to claim 16, **characterized in that** the method further comprises: introducing the spent catalyst generated in the fluidized bed reactor into the fluidized bed regenerator, introducing the regeneration gas into the regeneration zone of the fluidized bed regenerator to contact with the spent catalyst and react to produce the flue gas and the regenerated catalyst.

24. The use method according to claim 23, **characterized in that** the flue gas enters the regenerator gas-solid separation unit to remove regenerated catalyst entrained in it, then enters the regenerator gas collection chamber and is delivered to downstream sections through the flue gas delivery pipe.

25. The use method according to claim 23, **characterized in that** the method further comprises: the regenerated catalyst sequentially passes through the regenerator stripper and the regenerated catalyst slide valve to enter the riser reactor.

26. The use method according to claim 23, **characterized in that** the carbon content in the spent catalyst is in a range from 1.0 wt% to 3.0 wt%.

27. The use method according to claim 23, **characterized in that** the carbon content in the regenerated catalyst is ≤0.5 wt%.

28. The use method according to claim 23, **characterized in that** the regeneration gas is at least one selected from the group consisting of oxygen, air and oxygen-enriched air.

29. The use method according to claim 23, **characterized in that** the process conditions for the regeneration zone of the fluidized bed regenerator are: gas superficial velocity of 0.5-2.0 m/s, regeneration temperature of 600-750°C, regeneration pressure of 100-500 kPa, bed density of 150-700 kg/m³.

30. The use method according to claim 16, **characterized in that** the method further comprises: introducing the riser reactor feedstock and the catalyst into the riser reactor to react and produce aromatics;
the flow containing the unreacted riser reactor feedstock, aromatics and the catalyst enters the gas-solid separation unit II of the fluidized bed reactor from the outlet of the riser reactor.

31. The use method according to claim 30, **characterized in that** the catalyst is the regenerated catalyst from the fluidized bed regenerator.

32. The use method according to claim 30, **characterized in that** the riser reactor feedstock comprises water vapor and light alkanes separated from the product gas flow.

33. The use method according to claim 30, **characterized in that** the water vapor content in the riser reactor feedstock is in a range from 0 wt% to 50 wt%.

34. The use method according to claim 30, **characterized in that** the process conditions for the riser reactor are: gas superficial velocity of 3.0-10.0 m/s, temperature of 580-700°C, pressure of 100-500 kPa, bed density of 50-150 kg/m³.
